# EUROPEAN PATENT SPECIFICATION

(11) **EP 4 265 242 B1**
(45) Date of publication and mention of the grant of the patent: **03.09.2025**
(21) Application number: 23154522.9
(22) Date of filing: 01.02.2023
(51) Int. Cl.: A61K 9/50, A61K 31/195

(54) **HIGH DOSE TRANEXAMIC ACID COMPOSITION AND PROCESS FOR PREPARATION THEREOF**
HOCHDOSIERTE TRANEXAMSÄUREZUSAMMENSETZUNG UND VERFAHREN ZU IHRER HERSTELLUNG
COMPOSITION D'ACIDE TRANEXAMIQUE À DOSE ÉLEVÉE ET SON PROCÉDÉ DE PRÉPARATION

(30) Priority: 20.04.2022 IN 202211023210
(43) Date of publication of application: 25.10.2023
(73) Proprietor: Athena Pharmaceutiques SAS, 78430 Louveciennes (FR)
(72) Inventor: Chaudhari, Mahendra B., 400602 Naupada, Thane (West) Maharashtra (IN)
(74) Representative: Dr. Solf & Zapf Patent- und Rechtsanwalts PartG mbB

(56) References cited:
- US-A1- 2009 214 644
- US-B2- 8 597 683

## Description

### FIELD OF THE INVENTION

The present invention relates to a pharmaceutical formulation comprising high dose of tranexamic acid. In particular, the present invention relates to a particulate pharmaceutical formulation comprising high dose of active drug tranexamic acid for oral administration as well as a method for producing it for sachet formulation.

### BACKGROUND OF THE INVENTION

Tranexamic acid is chemically (trans-4-(aminomethyl) cyclohexane carboxylic acid. It has following chemical structure.

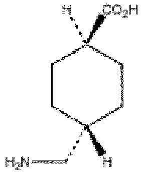

It is available commercially as Cyklokapron^{®} 500 mg oral tablet and Lysteda^{®} 650 mg oral tablet. Tranexamic acid is a white crystalline powder. It is freely soluble in water. It helps to prevent lysis or dissolution of a fibrin clot which forms in the normal physiologic process of hemostasis. Tranexamic acid thus helps to stabilize fibrin clots, which in turn maintains coagulation and helps to control bleeding. Lysteda^{®} is indicated for the treatment of heavy menstrual bleeding. The dosage regimen of Lysteda^{®} is 1,300 mg (two 650 mg tablets) three times a day (3,900 mg/day) for a maximum of 5 days during monthly menstruation. It is highly desirable to prepare a high dose composition to avoid the frequent dosing.

If 1 gm tranexamic acid is administered as 250 mg or 500 mg tablet, patient needs to swallow 4 or 2 tablets at a time. Alternatively, 1 gm tablet may be administered but with a drug load of about 50 %, the tablet will weigh about 2 gm making it difficult to swallow. Thus, there exists a need to provide a product which will allow administration of large dose of the drug without affecting patient compliance.

Accordingly, there is a need for a sachet dosage form comprising high dose of tranexamic acid which can contain high dose and can be consumed by the person in need thereof without any difficulty in swallowing.

US 2009/0214644 A1 discloses a delayed release oral dosage form comprising a core comprising tranexamic acid or a pharmaceutically acceptable salt thereof and a pharmaceutically acceptable excipient. The core is coated with a delayed release material which provides for the delayed release of the tranexamic acid or pharmaceutically acceptable salt thereof such that the dosage form is suitable for administration on a two or three times a day basis.

US 8 597 683 B2 discloses a modified release dosage form comprising a compressed matrix core which comprises tranexamic acid, a release controlling material, a binder, a filler, a glidant, a disintegrant, and optionally one or more lubricants. The dosage form further comprises a functional coating on the compressed matrix core which comprises a coating agent which is insoluble in water but soluble in aqueous media with a pH below 5, a film former, a plasticizer, and an anti-sticking agent.

### SUMMARY OF THE INVENTION

To achieve the foregoing and other objects and needs, the present invention provides a pharmaceutical formulation according to claim 1 comprising high dose of tranexamic acid. In particular, the present invention relates to a particulate pharmaceutical formulation comprising high dose of active drug tranexamic acid for oral administration as well as a method for producing it for sachet formulation.

According to the present invention, a high dose tranexamic acid composition comprises multi-layered particles, each particle comprising: i] an inert core; ii] a drug coating layer comprising tranexamic acid and povidone; and iii] a taste mask coating layer. The taste mask coating layer comprises: (a) a poly(meth)acrylate copolymer; (b) povidone; (c) sucralose; and (d) anhydrous colloidal silica. The tranexamic acid is in a weight ratio of 50 % to 60 % w/w of the composition.

In one or more embodiments, the inert core is in the form of sugar spheres.

In one or more embodiments, the inert core has a particle size of 200 microns (µm) - 600 microns (µm), and preferably from 355 microns (µm) - 500 microns (µm).

In one or more embodiments, the particles of the composition are in the form of pellets.

In one or more embodiments, the pellets are packaged into a sachet.

In one or more embodiments, the tranexamic acid is present in quantities of up to 2000 mg.

In one or more embodiments, the weight ratio of tranexamic acid: inert core is 1.5:1 to 3:1.

In one or more embodiments, the particle size of the composition is in the range of 250 microns (µm) to 1800 microns (µm), preferably from 400 microns (µm) to 1500 microns (µm) and more preferably from 600 microns (µm) to 1200 microns (µm).

In one or more embodiments, the poly(meth)acrylate copolymer used is Eudragit ^{®} NE 30D.

In one or more embodiments, the multi-layered composition further comprises a lubricant.

In another embodiment, the present invention relates to a process for preparing a high dose tranexamic acid composition comprising multi-layered particles. The process comprises the steps of: preparing a binder solution comprising povidone; layering tranexamic acid using the binder solution onto an inert core to form drug loaded pellets; preparing a taste mask coating suspension comprising poly(meth)acrylate copolymer, povidone, sucralose, and anhydrous colloidal silica; applying the taste mask coating suspension onto the drug loaded pellets; and lubricating the taste masked pellets. Thereby, the tranexamic acid is provided in a weight ratio of 50% to 60% w/w of the composition.

In one or more embodiments, the taste mask coating suspension is applied on to the drug loaded pellets in fluidized bed coater.

In one or more embodiments, tranexamic acid is present in quantities of upto 2000 mg.

In one or more embodiments, the weight ratio of tranexamic acid: inert core is 1.5:1 to 3:1.

In one or more embodiments, the particle size of the composition is in the range of 250 microns (µm) to 1800 microns (µm), preferably from 400 microns (µm) to 1500 microns (µm) and more preferably from 600 microns (µm) to 1200 microns (µm).

In one or more embodiments, the process further comprises filling the lubricated pellets into a sachet.

### BRIEF DESCRIPTION OF THE DRAWINGS

The advantages and features of the present invention will become better understood with reference to the following detailed description and claims taken in conjunction with the accompanying drawing, in which:
FIG. 1 illustrates a process flow for preparation of a multi-layered high dose tranexamic acid sachet composition, in accordance with an exemplary embodiment of the present invention;
FIG. 2 illustrates details for preparation of drug layering of process of FIG. 1;
FIG. 3 illustrates details for preparation of taste mask coating layer of process of FIG. 1;
FIG. 4 illustrates details for preparation of lubricated taste masked pellets of process of FIG. 1;
FIG. 5 illustrates details for filling the lubricated taste masked pellets into sachet; and
FIG. 6 illustrates summary of bioavailability study conducted between tranexamic acid sachet and reference product conducted as per Example 2.

### DETAILED DESCRIPTION OF THE INVENTION

The advantages and features of the present invention will become better understood with reference to the following detailed description and claims taken in conjunction with the accompanying drawing, in which:

The use of terms "including," "comprising," or "having" and variations thereof herein is meant to encompass the items listed thereafter and equivalents thereof as well as additional items. Further, the terms, "a" and "an" herein do not denote a limitation of quantity, but rather denote the presence of at least one of the referenced items.

Further, the terms, "a" and "an" herein do not denote a limitation of quantity, but rather denote the presence of at least one of the referenced items.

As used herein, "about" will be understood by persons of ordinary skill in the art and will vary to some extent depending upon the context in which it is used. If there are uses of the term in which are not clear to persons of ordinary skill in the art, given the context in which it is used, "about" will mean up to plus or minus 10 % of the particular term.

The term "multi-layered" means characterized by a well-designed architecture in which inert particles (or spherical carriers or pellets) are coated by one or more layers comprising tranexamic acid, on top of this by taste masking layer containing acrylate polymer. Details of this architecture as well as materials that can be used for it are explained below.

The present invention provides a high dose tranexamic acid composition which contains up to 2000 mg of tranexamic acid and has patient friendly dosing regimen. The Applicant has found that these characteristics can be obtained by formulating a sachet that contains tranexamic acid which is a multilayered composition and contains a mixture of excipients.

An advantageous embodiment relates to a multi-layered high dose tranexamic acid composition comprising: i] an inert core; ii] a drug coating layer that comprising tranexamic acid and a water soluble binder; and iii] a taste mask coating layer. The taste mask coating layer comprises: (a) a poly(meth)acrylate copolymer; (b) water soluble pore forming materials; (c) sweetener; and (d) anti-static agent.

As used herein, tranexamic acid includes free base as well as its pharmaceutically acceptable salts, enantiomers, polymorphic forms.

"Inert core" or "core material" is the one on which tranexamic acid is layered to make drug particles. The inert core according to the invention is a particle (or spherical carrier or pellet). The material used for the inert core is chemically inert, in the sense that it does not react with any of the other ingredients of the multi-layered particle according to the invention and especially does not interfere with the intended pharmacological mechanism exerted by the pharmaceutically active ingredient of the multi-layered particle. Examples for a material used for the inert core are cellulose, spheres of esp. microcrystalline cellulose, starch, lactose, sugar spheres, mannitol or mixtures thereof, or any other granular material such as granular mannitol, sugar crystals. Commercially available inert core materials can also be used. It has been found advantageous to use particles with a particle size (diameter) of about 200 µm to 600 µm, preferably with a small degree of variation. In one embodiment, the inert core has a particle size of 355 µm - 500 µm.

As explained below in more detail and demonstrated by the examples of this application, the multi-layered composition according to the invention is taste masked and at the same time they show advantageous dissolution profiles. Especially, the exemplified material poly(meth)acrylate turned out to be an extremely good outer coating layer which is insoluble in neutral pH environments (meal and mouth of the patient), but dissolves quickly at acidic pH (stomach), thereby allowing the particle to disintegrate and to release the drug. They further provide an acceptable mouth-feeling, i.e., no grittiness for the patient.

The percentage of core material in the final formulation (that is, the tranexamic acid composition) is varying from 10 % (w/w) to 50 % (w/w), more preferably from 15 % (w/w) to 40 % (w/w) and most preferably from 20 % (w/w) to 35 % (w/w) of the final formulation.

Pharmaceutically acceptable binders are soluble binders or insoluble binders. For soluble binders, water or hydro-alcoholic solvent is used as dissolving medium whereas for insoluble binders organic solvents are used as dissolving medium. Binders used in accordance with the present invention include but are not limited to tragacanth, acacia, gelatin, starch, cellulose materials such as methyl cellulose, microcrystalline cellulose and sodium carboxy methyl cellulose, hydroxypropyl cellulose, hydroxypropyl methylcellulose, methylcellulose, povidone, sucrose, alginic acids and salts thereof, polyethylene glycol, PVP, guar gum, polysaccharide acids, sugars, invert sugars and the like. In one embodiment, the water soluble binder used is povidone. Herein, the povidone use is polyvinyl pyrrolidone K30 (Kollidon **^{®}** 30).

Binders may be used in an amount of up to 20 % (w/w). In the tranexamic acid composition, the binders are present in an amount of up to 15 % (w/w), and preferably between 4 % (w/w) and 10 % (w/w).

The composition may be further coated with non-functional coating to prevent the drug loss from the drug particles during handling and sachet filling operation. The non-functional coating may also act as taste mask coating (hereinafter also interchangeably referred to as "taste mask coating layer" or "taste mask coating"). The taste mask coating layer includes poly(meth)acrylate copolymer (as a coating polymer), water soluble pore forming materials, sweetener and anti-static agent. The final formulation has this non-functional coating (taste mask coating) ranging between 3 % (w/w) to 5 % (w/w) of the final weight of the formulation.

The poly(meth)acrylate copolymer used are Eudragit^{®}E polymers that are methacrylic acid derivatives with a dimethylaminoethyl group. According to the fourth addition of the Handbook of Pharmaceutical Excipients, Eudragit **^{®}** E is a cationic polymer based on dimethylaminoethyl methacrylate and other neutral methacrylic acid esters. It is soluble in gastric fluid as well as in weakly acidic buffer solutions (up to pH of approximately 5). In one embodiment, the poly(meth)acrylate copolymer used is Eudragit **^{®}** NE 30D. In the tranexamic acid composition, the poly(meth)acrylate copolymer is present in an amount between 2 % (w/w) and 4 % (w/w).

The water soluble pore forming materials include polymeric materials such as povidone, hydroxyl propyl methyl cellulose, hydroxyl propyl cellulose, sugar, or acrylate polymers. In one embodiment, the water soluble pore forming material is povidone. In the tranexamic acid composition, the water soluble pore forming materials are present in an amount between 0.25 % (w/w) and 0.75 % (w/w)*.*

"Palatability" and "mouth feel" are among the most important characteristics to be considered in providing sachet dosage forms, for a drug. Unfortunately, many drugs have a bitter or otherwise unpalatable taste, or an unacceptable mouth feel, which make such drugs unsuitable for administration as sachet. Much research has been devoted to designing techniques and approaches to mask the bitter taste of drug in dosage forms. Simple approaches include adding chemicals mediating, flavoring or sweetening ingredients to the composition, which thereby mask the bitterness of the drug.

Anhydrous colloidal silica is used as an antistatic agent. The antistatic agents include, but are not limited to, micronized and non- micronized talc, fumed silica (Aerosil ^{®} R 972), colloidal silica (Aerosil ^{®} 200), precipitated silica (Syloid ^{®} FP 244), and combination thereof. Antistatic agent is present in the formulation in the range of 0.5 to 5.0 % of the final tablet weight. Antistatic agent is incorporated in the granules to improve the flow of the material. In a specific example, the antistatic agent is Aerosil ^{®} 200. In the tranexamic acid composition, the anhydrous colloidal silica is present in an amount between 0.2 % (w/w) and 0.6 % (w/w)*.*

Besides the above materials, the taste masking layer may include one or more pharmaceutically inert excipients such as sweeteners, solvents, lubricants/glidants, plasticizers and preservatives which are well known in the art of pharmaceutical formulations.

Sweeteners play an important role in developing palatable taste-masked formulations. They can help reduce bitterness but are most effective when combined with buffers for sourness and sodium salts for saltiness. The sweeteners in include dextrose, fructose, sucrose, erythritol, maltitol, mannitol, sorbitol, xylitol, aspartame, saccharin, sodium saccharin, sucralose and the like. In one embodiment, the sweetener used is sucralose. In the tranexamic acid composition, the sweeteners are used in the range of 0.05 % (w/w) to 0.5 % (w/w)*.*

The tranexamic acid may additionally comprise lubricants. Lubricants may be selected from, but are not limited to, those conventionally known in the art such as Mg (magnesium), Al (aluminum) or Ca (calcium) or Zn (zinc) stearate, sodium stearyl fumarate, stearic acid, sodium benzoate, micronized macrogols, and other materials known to one ordinarily skilled in the art and combinations thereof. The amount of lubricant is from 0 % (w/w) to 3 % (w/w), preferably from 0 % (w/w) to 2 % (w/w) and more preferably 1 % (w/w). In one embodiment, lubricant used in the present invention is purified Talc.

In an embodiment, the present invention relates to a multi-layered high dose tranexamic acid composition comprising: i] an inert core; ii] a drug coating layer that comprising tranexamic acid and povidone; and iii] a taste mask coating layer. The taste mask coating layer comprises: (a) a poly(meth)acrylate copolymer; (b) povidone; (c) sucralose; and (d) anhydrous colloidal silica.

The composition as per the present invention involves fluidized bed coating, a process by which coated particles are produced in a single piece of equipment by spraying a binder as solution, suspension, or melt onto a fluidized powder bed. This process is sometimes classified as the one-pot system. It provides several advantages like uniform coating; good process control and good edge coverage.

In an aspect of the embodiment, the weight ratio of tranexamic acid: inert core is 1.5:1 to 3:1.

In an aspect of the embodiment, the multi-layered composition has particle size in the range of 250 microns (µm) to 1800 microns (µm), preferably in the range of 400 microns (µm) to 1500 microns (µm) and more preferably from 600 microns (µm) to 1200 microns (µm).

According to the invention, tranexamic acid is present in a weight ratio of 50 % to 60 % w/w of the composition.

In another embodiment, the present invention relates to a process for preparing multi-layered high dose tranexamic acid composition. The process comprising the steps of: preparing a binder solution comprising povidone; layering tranexamic acid using the binder solution onto an inert core to form drug loaded pellets; preparing a taste mask coating suspension comprising poly(meth)acrylate copolymer, povidone, sucralose and anhydrous colloidal silica; applying the taste mask coating suspension on to the drug loaded pellets; and lubricating the taste masked pellets.

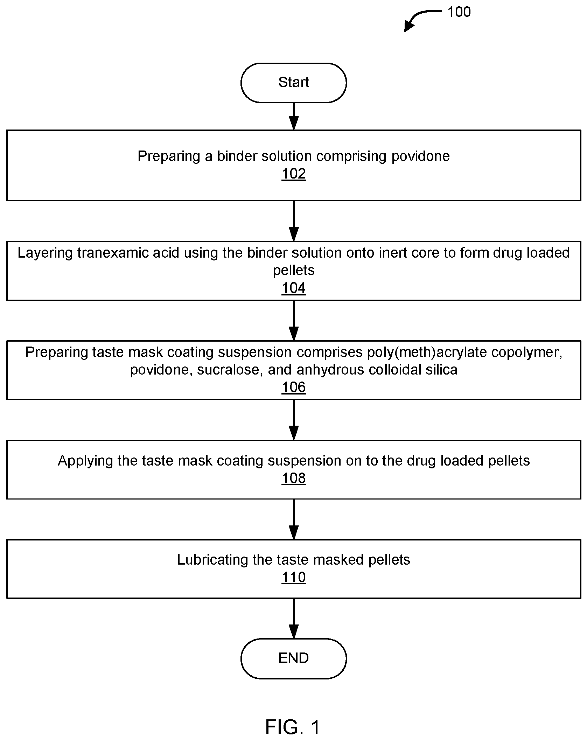

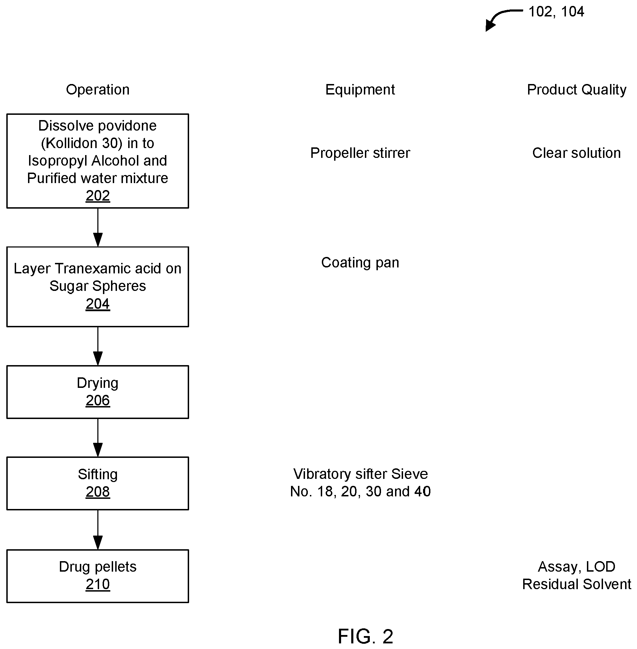

Referring to FIG. 1, at step 102, the process 100 comprises preparing a binder solution comprising povidone. At step 104, the process 100 comprises layering tranexamic acid using the binder solution onto inert core to form drug loaded pellets. The process 100 is initiated by preparing a binder solution comprising povidone. The step 102 and step 104 are described in detail in FIG. 2. Referring to FIG. 2, at step 202, povidone (Kollidon ^{®} 30) is dissolved in to isopropyl alcohol and purified water mixture under continuous stirring till clear solution resulted. The final solution is filtered through 60# / nylon cloth to get the binder solution.

Next at step 204, the tranexamic acid is layered using the binder solution loading suspension onto an inert core to form drug loaded pellets. Specifically, the binder solution is sprayed continuously sprayed on sugar spheres (that is, the inert core) in a revolving coating pan. Tranexamic acid is intermittently added in the pan and mixed well to distribute it and adhere to sugar spheres. Once the entire tranexamic acid is adhered on the sugar spheres, then the sugar spheres are dried at step 206 in the revolving coating pan in hot air at pre-40°C till loss on drying of the pellets is less than 3%, to obtain dried drug pellets. Then, at step 208, the drug pellets are sifted through 1000 microns screen to remove agglomerate mass. The pellets are resifted through 500 microns screen to remove fine powder. Thereafter, at step 210, the drug pellets are obtained. In preferred embodiments, the layering or coating is done in a solid coating pan or in a fluid bed coater or using extrusion and spheronisation.

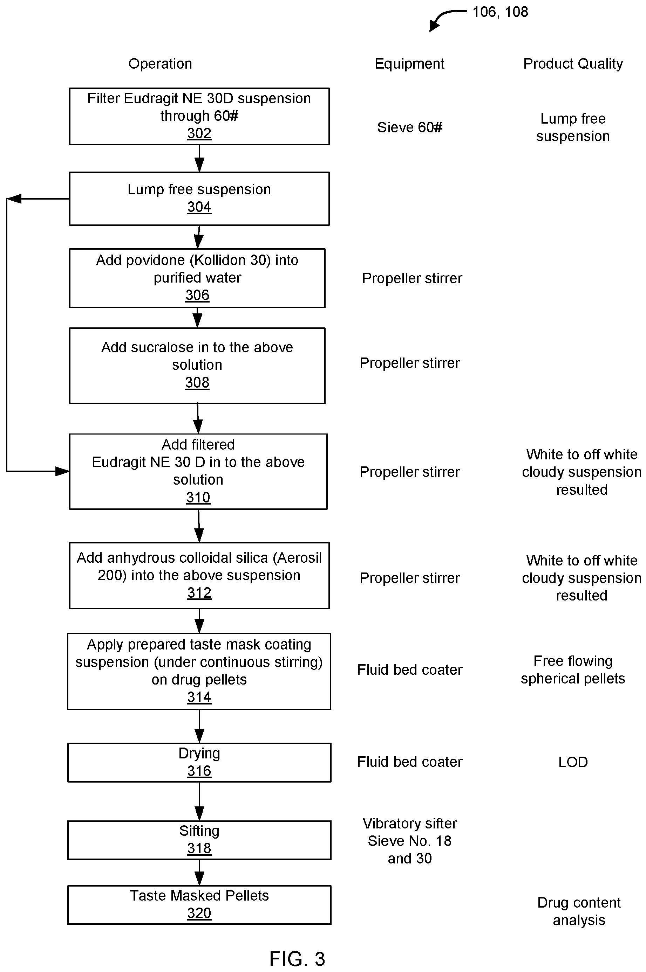

Again, referring to FIG. 1, at step 106, the next step in the process 100 comprises preparing taste mask coating suspension comprises poly(meth)acrylate copolymer, povidone, sucralose, and anhydrous colloidal silica. The step 106 is described in detail in FIG. 3. Referring to FIG. 3, at step 302, Eudragit^{®} NE 30 D suspension is filtered through 60 mesh to form a lump free suspension at step 304. Simultaneously, a suitable SS tank with stirrer is taken in which purified water is added and thereafter at step 306, povidone is added under continuous stirring to form a solution. Thereafter, at step 308, sucralose is added into the above solution and dissolved by continuous stirring. Thereafter, at step 310, the previously filtered Eudragit ^{®} NE 30 D suspension at step 302 is added to the above solution obtained at step 308, and continuously stirred till get white to off cloudy suspension resulted. Thereafter, at step 312, anhydrous colloidal silica (for example, Aerosil ^{®} 200) is added into the above solution obtained at step 310, and dissolved by stirring process till get white to off cloudy suspension resulted. At this step (that is, 312), taste mask coating suspension is obtained.

Again, referring to FIG. 1, at step 108, the next step in the process 100 comprises applying the taste mask coating suspension on to the drug loaded pellets. The step 108 is described in detail in FIG. 3. Referring again to FIG. 3, at step 314, the prepared taste mask coating suspension is applied on to the drug pellets in fluidized bed coater by continuous stirring process at pre-determined parameters. As used herein, in a non-limiting example, pre-determined parameters include an inlet temperature of about 30°C to about 50°C, product temperature of about 25°C to about 35°C, damper opening of about 30 % to 90 %, spraying rate of the prepared taste mask coating suspension of about 200 g/min to about 500 g/min and atmospheric pressure of about 1.0 kg/cm² to about 3.0 kg/cm² to obtain taste masked pellets. Although particular pre-determined parameters are mentioned above, it will be evident to a person skilled in the art to modify predetermined parameters based on obvious variations.

Next, at step 316, the taste masked pellets are dried in the fluidized bed coater at the pre-determined parameters for 1 hour. As used herein, in a non-limiting example, pre-determined parameters include an inlet temperature of about 30°C to about 50°C, product temperature of about 25°C to about 35°C, damper opening of about 30 % to 90 %. Although particular pre-determined parameters are mentioned above, it will be evident to a person skilled in the art to modify predetermined parameters based on obvious variations. Thereafter, at step 318, the dried drug pellets are sifted and sieved through 18 mesh fitted on the vibratory sifter and remove the agglomerate pellets. Further, the drug pellets are resifted through 30 mesh screen fitted on vibratory sifter and remove the fine pellets. The sifted pellets (below 18 mesh) are collected in HDPE container lined with polybag and weigh the sifted pellets. Finally, at step 320, the taste masked pellets are obtained after sifting process.

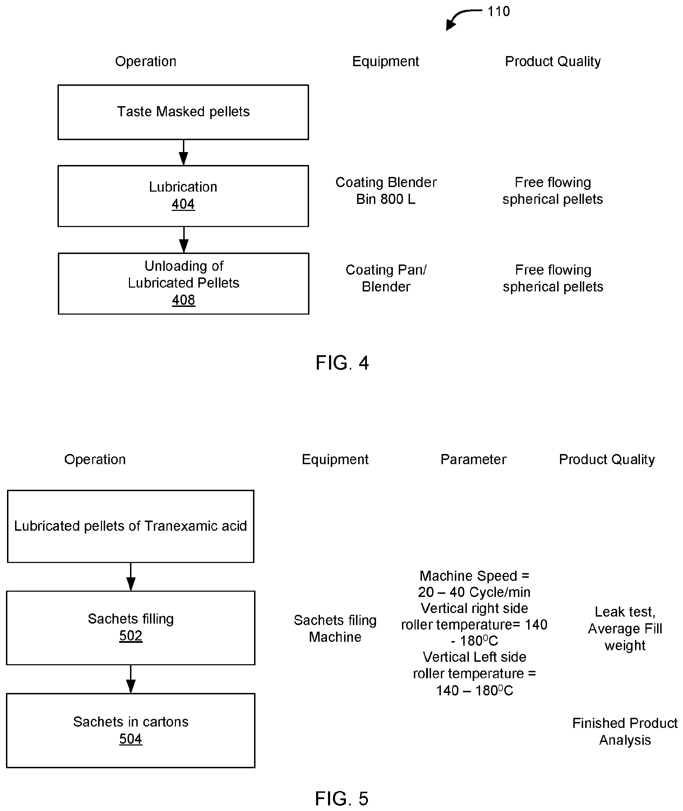

Now referring again to FIG. 1, at step 110, the process 100 comprises lubricating the taste masked pellets. The step 110 is described in detail in FIG. 4. Next, at step 402, the taste masked pellets are lubricated using talc in conta blender bin 800 L and mix the taste masked pellets for about 5 minutes to form lubricated taste masked pellets. Thereafter at step 404, the lubricated taste masked pellets are unloaded into suitable drums labeled with product name batch no and weights and transfer to pellets storage area.

Now referring again to FIG. 5, at step 502, the lubricated taste masked pellets are filled or packaged into sachets by using sachet filling machine. Finally, at step 504, filled sachets are manually placed in cartons for finished product analysis.

Conventional instruments like blenders, sifters, coating instruments, and the sachet machine are used for the manufacturing of high dose of Tranexamic acid.

The description of the present invention of pharmaceutical composition and process for preparation thereof is further illustrated by the following non-limiting example. A person skilled in the art would recognize that, the specific example is intended to illustrate, not limit, the scope of the present invention.

### EXAMPLE 1

In Example 1, a process for preparing a tranexamic acid composition is described. Particularly, the process is described for preparing the tranexamic acid composition having a strength of 1000 mg per sachet. Herein, tranexamic acid is in a weight ratio of 50 % to 60 % w/w of the composition.

The process is initiated by preparing a binder solution comprising povidone. 108.85 mg of povidone (Kollidon ^{®} 30) is dissolved in to isopropyl alcohol and purified water mixture. The final solution is filtered through 60# / nylon cloth to get the binder solution.

The next step is layering the tranexamic acid using the binder solution loading suspension onto an inert core to form drug loaded pellets. Specifically, the binder solution is sprayed continuously sprayed on 567.65 mg of sugar spheres (that is, the inert core) in a revolving coating pan. 1000 mg of tranexamic acid is intermittently added in the pan and mixed well to distribute it and adhere to sugar spheres. Once the entire tranexamic acid is adhered on the sugar spheres, then the sugar spheres are dried in the revolving coating pan in hot air at pre-40°C till loss on drying of the pellets is less than 3%, to obtain dried drug pellets. Then, the drug pellets are sifted through 1000 microns screen to remove agglomerate mass. The pellets are resifted through 500 microns screen to remove fine powder. In preferred embodiments, the layering or coating is done in a solid coating pan or in a fluid bed coater or using extrusion and spheronisation.

In TABLE 1A below, the different ingredients for performing drug layering on sugar spheres which includes preparation of binder solution, drug layering in coating pan, drying, and sifting and sizing, are depicted.

**TABLE 1A**

| Ingredients | Solution | Qty. (mg) for 1000 mg (1 g) of tranexamic acid per sachet |
|---|---|---|
| Tranexamic acid | Active | 1000.0 |
| Sugar spheres (400 µm - 600 µm) | Core | 567.65 |
| Povidone (Kollidon 30) | Binder | 108.85 |
| Isopropyl Alcohol | Solvent | Q.S |
| Purified water | Solvent | Q.S |

In the next step of the process, the taste mask coating suspension is prepared comprising poly(meth)acrylate copolymer, povidone, sucralose and anhydrous colloidal silica. Particularly, 52.50 mg of Eudragit ^{®} NE 30 D suspension is filtered through 60 mesh to form a lump free suspension. Simultaneously, a suitable SS tank with stirrer is taken in which purified water is added and thereafter 8.75 mg of povidone is added under continuous stirring to form a solution. Thereafter, 1.75 mg of sucralose is added into the above solution and dissolved by continuous stirring. Thereafter, to the above solution, the previously filtered Eudragit ^{®} NE 30 D suspension is added and continuously stirred. Thereafter 7 mg of anhydrous colloidal silica (for example, Aerosil ^{®} 200) is added into the above solution and dissolved by stirring process.

In TABLE 1B, the different ingredients for preparing taste mask coating are depicted.

**TABLE 1B**

| Ingredients | Solution | Qty. (mg) for 1000 mg (1 g) of tranexamic acid per sachet |
|---|---|---|
| Eudragit NE 30D | Coating polymer | 52.50 |
| Povidone (Kollidon 30) | Pore forming agent | 17.5 |
| Sucralose | Sweetener | 3.5 |
| Anhydrous colloidal silica (Aerosil 200) | Anti-static agent | 7.0 |
| Purified water | Solvent | Q.S |

Thereafter, the prepared taste mask coating suspension is applied on to the drug loaded pellets in fluidized bed coater by continuous stirring process at pre-determined parameters. As used herein, in a non-limiting example, pre-determined parameters include an inlet temperature of about 30°C to about 50°C, product temperature of about 25°C to about 35°C, damper opening of about 30 % to 90 %, spraying rate of the prepared taste mask coating suspension of about 200 g/min to about 500 g/min and atmospheric pressure of about 1.0 kg/cm² to about 3.0 kg/cm² to obtain taste masked pellets. Although particular pre-determined parameters are mentioned above, it will be evident to a person skilled in the art to modify predetermined parameters based on obvious variations.

Thereafter, the taste masked pellets are dried in the fluidized bed coater at the pre-determined parameters for 1 hour. As used herein, in a non-limiting example, pre-determined parameters include an inlet temperature of about 30°C to about 50°C, product temperature of about 25°C to about 35°C, damper opening of about 30 % to 90 %. Although particular pre-determined parameters are mentioned above, it will be evident to a person skilled in the art to modify predetermined parameters based on obvious variations. Finally, the dried taste masked pellets are sifted in a vibratory sifter to obtain taste masked pellets.

In the next step of process, the taste masked pellets are lubricated. The taste masked pellets are lubricated with 3.50 mg of purified talc in a coating pan/ blender for about 5 minutes to form lubricated taste masked pellets.

In TABLE 1C, the different ingredients for lubricating the taste masked pellets with purified talc are depicted.

**TABLE 1C**

| Ingredients | Solution | Qty. (mg) for 1000 mg of tranexamic acid per sachet |
|---|---|---|
| Purified Talc | Lubricant | 3.5 |

The following table provides a percentage by weight of the composition of the high dose of tranexamic acid (1000 mg).

**TABLE 2**

| Ingredients | Relative Qty. |
|---|---|
| | % w/w |
| Tranexamic acid | 57.14 |
| Sugar spheres (400 µm - 600 µm) | 32.44 |
| Povidone (Kollidon 30) | 6.22 |
| Isopropyl Alcohol | Q.S |
| Purified Water | Q.S |
| Eudragit NE 30D | 3.00 |
| Povidone (Kollidon 30) | 0.50 |
| Sucralose | 0.10 |
| Anhydrous colloidal silica (Aerosil 200) | 0.40 |
| Purified Talc | 0.20 |
| Purified Water | Q.S |
| Total | 100.0 |

The following table provides actual quantity of pellets in kg and actual quantity of pellets for 125000 sachets in kg.

**TABLE 3**

| Ingredients | mg/sachet | Actual Qty. (Kg) of pellets | Actual Qty. (Kg) of pellets for 125000 sachets |
|---|---|---|---|
| Tranexamic acid | 1000.0 | 128.57 | 125.00 |
| Sugar Spheres (400 µm - 600 µm) | 567.65 | 72.98 | 70.96 |
| Povidone (Kollidon 30) | 108.85 | 14.00 | 13.61 |
| Isopropyl Alcohol | Q.S | 59.40 | 57.75 |
| Purified Water | Q.S | 6.60 | 6.42 |
| Eudragit NE 30D | 52.50 | 6.75 | 6.56 |
| Povidone (Kollidon 30) | 8.75 | 1.12 | 1.09 |
| Sucralose | 1.75 | 0.23 | 0.22 |
| Anhydrous colloidal silica (Aerosil 200) | 7.00 | 0.90 | 0.87 |
| Purified Water | Q.S | 35.25 | 34.27 |
| Purified Talc | 3.50 | 0.45 | 0.44 |
| Total | 1750.0 | 225.0 | 218.75 |

### EXAMPLE 2

In Example 2, a Bioavailability study was conducted between the Test product: tranexamic acid 1 g coated granules in Sachet and reference product: Cyklo-f 500 mg film coated tablet, Meda AB, Sweden. An Open Label, Balanced, Randomized, Single-Dose, Two-Treatment, Two-Period, Two-Sequence, Two-Way Crossover, Comparative Bioavailability Study of tranexamic acid granules in sachets (1 g) of Athena Drug Delivery Solutions Pvt. Ltd., India with Cyklo-f 500 mg Film coated tablet of Meda AB, Box 906, 170 09, Solna in Healthy, Adult, Human Subjects Under Fasting Conditions.

Pharmacokinetic parameters of Test product tranexamic acid coated granules 1 g sachet are bioequivalent to the Pharmacokinetic parameters of 2 tablets of reference drug tranexamic acid 500 mg tablet in this study.

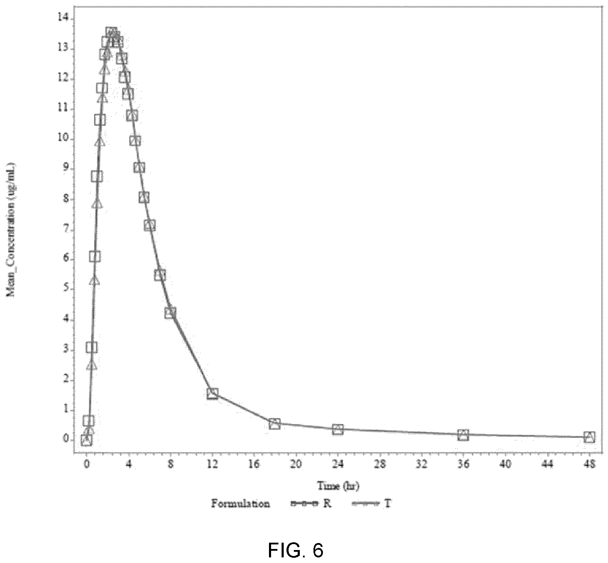

FIG. 6 illustrates summary of bioavailability study conducted between tranexamic acid sachet and reference product conducted as per Example 2.

**TABLE 4 - The following table provides summary of the study.**

| **Parameters** | **% T/R ratio** | **Intra Subject Variability** | **Acceptance Range** | **90% CI** | **Power** |
|---|---|---|---|---|---|
| Cₘₐₓ | 99.88 | 13.85 | 80.00 - 125.00 | 94.20 - 105.91 | 100 |
| AUC₀₋ₜ | 99.42 | 14.47 | 80.00 - 125.00 | 93.52 - 105.70 | 100 |

Accordingly, pharmacokinetic parameters of Test drug tranexamic acid granules 1 g sachet are bioequivalent to the Pharmacokinetic parameters of 2 tablets of reference drug tranexamic acid 500 mg tablet.

## Claims

1. A high dose tranexamic acid composition comprising multi-layered particles,
each particle comprising:
i] an inert core;
ii] a drug coating layer comprising tranexamic acid and povidone; and
iii] a taste mask coating layer comprising
(a) a poly(meth)acrylate copolymer;
(b) povidone;
(c) sucralose; and
(d) anhydrous colloidal silica,
wherein tranexamic acid is in a weight ratio of 50% to 60% w/w of the composition.

2. The composition as claimed in Claim 1,
**characterized in that**
the inert core is in the form of sugar spheres.

3. The composition as claimed in Claim 1 or 2,
**characterized in that**
the inert core has a particle size of 200 microns (µm) - 600 microns (µm), and preferably from 355 microns (µm) - 500 microns (µm).

4. The composition as claimed in one of the previous Claims,
**characterized in that**
the multi-layered particles of the composition are in the form of pellets.

5. The composition as claimed in one of the previous Claims,
**characterized in that**
the plurality of the multi-layered particles as a whole comprises tranexamic acid in quantities of up to 2000 mg.

6. The composition as claimed in one of the previous Claims,
**characterized in that**
the weight ratio of tranexamic acid : inert core is 1.5:1 to 3:1.

7. The composition as claimed in one of the previous Claims,
**characterized in that**
the particle size of the multi-layered particles of the composition is in the range of 250 microns (µm) to 1800 microns (µm), preferably from 400 microns (µm) to 1500 microns (µm), and more preferably from 600 microns (µm) to 1200 microns (µm).

8. The composition as claimed in one of the previous Claims,
**characterized in that**
the poly(meth)acrylate copolymer used is Eudragit ^{®} NE 30D.

9. The composition as claimed in one of the previous Claims,
**characterized in that**
the composition further comprises a lubricant.

10. A sachet,
**characterized in that**
the sachet comprises the composition as claimed in one of the previous Claims.

11. A process for preparing a high dose tranexamic acid composition comprising multi-layered particles, preferably a composition as claimed in one of Claims 1 to 9, the process comprising the steps of:
- preparing a binder solution comprising povidone;
- layering tranexamic acid using the binder solution onto an inert core to form drug loaded pellets;
- preparing a taste mask coating suspension comprising poly(meth)acrylate copolymer, povidone, sucralose and anhydrous colloidal silica;
- applying the taste mask coating suspension onto the drug loaded pellets; and
- lubricating the taste masked pellets,
wherein tranexamic acid is provided in a weight ratio of 50% to 60% w/w of the composition.

12. The process as claimed in Claim 11,
**characterized in that**
the taste mask coating suspension is applied on to the drug loaded pellets in a fluidized bed coater.

13. The process as claimed in Claim 11 or 12,
**characterized in that**
the process comprises filling the lubricated pellets into a sachet.

## Patentansprüche

1. Hochdosierte Tranexamsäurezusammensetzung, die mehrschichtige Partikel aufweist, wobei jedes Partikel aufweist:
i] einen inerten Kern;
ii] eine Wirkstoffbeschichtung, die Tranexamsäure und Povidon aufweist; und
iii] eine Geschmacksmaskierungsschicht, die aufweist
(a) ein Poly(meth)acrylat-Copolymer;
(b) Povidon;
(c) Sucralose; und
(d) wasserfreies kolloidales Siliciumdioxid,
wobei Tranexamsäure in einem Gewichtsanteil von 50 Gew.-% bis 60 Gew.-% der Zusammensetzung enthalten ist.

2. Zusammensetzung nach Anspruch 1,
**dadurch gekennzeichnet, dass**
der inerte Kern in Form von Zuckerkugeln vorliegt.

3. Zusammensetzung nach Anspruch 1 oder 2,
**dadurch gekennzeichnet, dass**
der inerte Kern eine Partikelgröße von 200 Mikrometern (µm) bis 600 Mikrometern (µm), und vorzugsweise von 355 Mikrometern (µm) bis 500 Mikrometern (µm), aufweist.

4. Zusammensetzung nach einem der vorhergehenden Ansprüche,
**dadurch gekennzeichnet, dass**
die mehrschichtigen Partikel der Zusammensetzung in Form von Pellets vorliegen.

5. Zusammensetzung nach einem der vorhergehenden Ansprüche,
**dadurch gekennzeichnet, dass**
die Gesamtheit der mehrschichtigen Partikel Tranexamsäure in Mengen von bis zu 2000 mg aufweist.

6. Zusammensetzung gemäß einem der vorhergehenden Ansprüche,
**dadurch gekennzeichnet, dass**
das Gewichtsverhältnis von Tranexamsäure zu inertem Kern 1,5:1 bis 3:1 beträgt.

7. Zusammensetzung gemäß einem der vorhergehenden Ansprüche,
**dadurch gekennzeichnet, dass**
die Partikelgröße der mehrschichtigen Partikel der Zusammensetzung im Bereich von 250 Mikrometern (µm) bis 1800 Mikrometern (µm), vorzugsweise von 400 Mikrometern (µm) bis 1500 Mikrometern (µm) und noch bevorzugter von 600 Mikrometern (µm) bis 1200 Mikrometern (µm) liegt.

8. Zusammensetzung nach einem der vorhergehenden Ansprüche,
**dadurch gekennzeichnet, dass**
das verwendete Poly(meth)acrylat-Copolymer Eudragit^{®} NE 30D ist.

9. Zusammensetzung nach einem der vorhergehenden Ansprüche,
**dadurch gekennzeichnet, dass**
die Zusammensetzung ferner ein Gleitmittel umfasst.

10. Beutel,
**dadurch gekennzeichnet, dass**
der Beutel die Zusammensetzung gemäß einem der vorhergehenden Ansprüche aufweist.

11. Verfahren zur Herstellung einer hochdosierten Tranexamsäurezusammensetzung, die mehrschichtige Partikel aufweist, vorzugsweise einer Zusammensetzung gemäß einem der Ansprüche 1 bis 9,
wobei das Verfahren die folgenden Schritte aufweist:
- Herstellen einer Bindemittellösung, die Povidon umfasst;
- Aufbringen von Tranexamsäure unter Verwendung der Bindemittellösung auf einen inerten Kern, um mit Wirkstoff beladene Pellets zu bilden;
- Herstellen einer Geschmacksmaskierungsbeschichtungssuspension, die Poly(meth)acrylat-Copolymer, Povidon, Sucralose und wasserfreies kolloidales Siliciumdioxid aufweist;
- Auftragen der Geschmacksmaskierungsbeschichtungssuspension auf die mit Wirkstoff beladenen Pellets; und
- Schmieren der geschmacksmaskierten Pellets,
wobei Tranexamsäure in einem Gewichtsanteil von 50 Gew.-% bis 60 Gew.-% der Zusammensetzung bereitgestellt wird.

12. Verfahren gemäß Anspruch 11,
**dadurch gekennzeichnet, dass**
die Geschmacksmaskierungsbeschichtungssuspension in einem Fließbettbeschichter auf die mit Wirkstoff beladenen Pellets aufgebracht wird.

13. Verfahren nach Anspruch 11 oder 12 ,
**dadurch gekennzeichnet, dass**
das Verfahren das Einfüllen der geschmierten Pellets in einen Beutel umfasst.

## Revendications

1. Composition à haute dose d'acide tranexamique comprenant des particules multicouches, chaque particule comprenant:
i] un noyau inerte;
ii] une couche d'enrobage médicamenteuse comprenant de l'acide tranexamique et de la povidone; et
iii] une couche d'enrobage masquant le goût comprenant
(a) un copolymère de poly(méth)acrylate;
(b) de la povidone;
(c) du sucralose; et
(d) de la silice colloïdale anhydre,
où l'acide tranexamique est présent dans une quantité de 50 % à 60 % en poids de la composition.

2. La composition selon la revendication 1,
**caractérisée en ce que**
le noyau inerte se présente sous la forme de sphères de sucre.

3. La composition selon la revendication 1 ou 2,
**caractérisée en ce que**
le noyau inerte a une taille de particules comprise entre 200 microns (µm) et 600 microns (µm), et de préférence entre 355 microns (µm) et 500 microns (µm).

4. La composition selon l'une des revendications précédentes,
**caractérisée en ce que**
les particules multicouches de la composition se présentent sous forme de pellets.

5. La composition selon l'une des revendications précédentes,
**caractérisée en ce que**
la pluralité de particules multicouches dans son ensemble comprend de l'acide tranexamique en quantités allant jusqu'à 2000 mg.

6. La composition selon l'une des revendications précédentes,
**caractérisée en ce que**
le rapport pondéral entre l'acide tranexamique et le noyau inerte est compris entre 1,5:1 et 3:1.

7. La composition selon l'une des revendications précédentes,
**caractérisée en ce que**
la taille des particules multicouches de la composition est comprise entre 250 microns (µm) et 1800 microns (µm), de préférence entre 400 microns (µm) et 1500 microns (µm), et plus préférablement entre 600 microns (µm) et 1200 microns (µm).

8. La composition selon l'une des revendications précédentes,
**caractérisée en ce que**
le copolymère de poly(méth)acrylate utilisé est l'Eudragit^{®} NE 30D.

9. La composition selon l'une des revendications précédentes,
**caractérisée en ce que**
la composition comprend en outre un lubrifiant.

10. Sachet,
**caractérisé en ce que**
le sachet comprend la composition selon l'une des revendications précédentes.

11. Procédé de préparation d'une composition à haute dose d'acide tranexamique comprenant des particules multicouches, de préférence d'une composition selon l'une des revendications 1 à 9, le procédé comprenant les étapes suivantes:
- préparation d'une solution de liant comprenant de la povidone;
- la mise en couche de l'acide tranexamique à l'aide de la solution de liant sur un noyau inerte pour former des pellets chargés en médicament;
- préparation d'une suspension de revêtement masquant le goût comprenant du copolymère de poly(méth)acrylate, de la povidone, du sucralose et de la silice colloïdale anhydre;
- appliquer la suspension de revêtement masquant le goût sur les pellets chargés en médicament; et
- lubrifier les pellets masqués de goût,
où l'acide tranexamique est mis à disposition dans une quantité de 50 % à 60 % en poids de la composition.

12. Le procédé selon la revendication 11,
**caractérisé en ce que**
la suspension de revêtement masquant le goût est appliquée sur les pellets chargés en médicament dans un enrobeur à lit fluidisé.

13. Le procédé selon la revendication 11 ou12,
**caractérisé en ce que**
le procédé comprend le remplissage des pellets lubrifiés dans un sachet.
